# EUROPÄISCHE PATENTSCHRIFT

(11) **EP 0 998 974 B1**
(45) Veröffentlichungstag und Bekanntmachung des Hinweises auf die Patenterteilung: **25.06.2003**
(21) Anmeldenummer: 99121693.8
(22) Anmeldetag: 29.10.1999
(51) Int. Cl.: B01J 19/00, C12Q 1/68, G01N 33/543

(54) **Verfahren zum gezielten Aufbringen von Reagenzien auf immobilisiertes biologisches Material**
Method for the targetted application of reagents to immobilised biological material
Méthode pour l'application ponctuelle des réactifs à matière biologique immobilisée

(30) Priorität: 03.11.1998 DE 19850659
(43) Veröffentlichungstag der Anmeldung: 10.05.2000
(73) Patentinhaber: MetaSystems Hard & Software GmbH, 68804 Altlussheim (DE)
(72) Erfinder: Lörch, Thomas, Dr., 68799 Reilingen (DE); Plesch, Andreas, Dr., 68723 Schwetzingen (DE); Haas, Oskar A., Prof., 3400 Kloster Neuburg (AT)
(74) Vertreter: Müller-Boré & Partner Patentanwälte

(56) Entgegenhaltungen:
- EP-A- 0 834 575
- WO-A-95/12808
- WO-A-97/26539
- DE-A- 4 344 726
- O.-P. KALLIONIEMI ET AL.: "Optimizing comparative genomic hybridization for analysis of DNA sequence copy number changes in solid tumours." GENES, CHROMOSOMES & CANCER, Bd. 10, Nr. 4, August 1994 (1994-08), Seiten 231-243, XP000606237

## Beschreibung

Die vorliegende Erfindung betrifft ein Verfahren zum gezielten Aufbringen von mindestens einem Reagenz auf immobilisiertes biologisches Material.

Moderne Methoden zur Untersuchung immobilisierten biologischen Materials wie z.B. Zellen oder Chromosomen verwenden häufig teure Reagenzien, bei denen beispielsweise Fluorochrome zur Markierung eingesetzt werden. Beispiele solcher Methoden sind u. a. FISH- (Fluoreszenz*-in-situ*-Hybridisierung) bzw. Multi-Color-FISH-Verfahren, wobei die Zellen z.B. mit Fluoreszenz-markierten Nukleinsäure-Sonden oder -Sondengemischen hybridisiert werden. Ein Anwendungsbeispiel solcher FISH-Methoden ist die Metaphasenanalyse. Nur ein kleiner Teil der dabei eingesetzten DNA-Sonden kann für die Analyse genutzt werden, da die Metaphasechromosomen nur einen kleinen Anteil des hybridisierten Materials beziehungsweise der hybridisierten Fläche ausmachen. Der größte Teil der DNA-Sonden bleibt daher ungenutzt und wird abgewaschen und verworfen.

Ein Ausweg aus dieser Situation ist die gezielte Aufbringung von Reagenzien auf Bereiche eines Trägers wie z.B. einem Objektträger, auf dem das interessierende biologische Material, im Falle der Metaphasenanalyse also die Metaphasenchromosomen, immobilisiert ist, etwa durch gezieltes Auftropfen entsprechend kleiner Flüssigkeitsvolumina. Das teure Ausgangsmaterial der Reagenzien wie z.B. Fluoreszenz-markierte DNA-Sonden kann so maximal genutzt werden.

DE 43 44 726 A1 offenbart eine Anordnung von Nukleinsäuresequenzen, dadurch gekennzeichnet, daß alle Komponenten dieser Anordnung von Nukleinsäuresequenzen von einander getrennt sind.

WO 97 26539 A offenbart ein System, um die Anwesenheit eines Zielbiomoleküls in einer Probe nachzuweisen.

Somit liegt der vorliegenden Erfindung die Aufgabe zugrunde, ein Verfahren zum gezielten Aufbringen von mindestens einem Reagenz auf immobilisiertes biologisches Material bereitzustellen, das eine verbesserte Ausnutzung der aufzubringenden Reagenzien ermöglicht.

Diese Aufgabe wird durch die in den Ansprüchen gekennzeichneten Ausführungsformen der vorliegenden Erfindung gelöst.

Insbesondere wird ein Verfahren zum gezielten Aufbringen von mindestens einem Reagenz auf biologisches Material, das auf einem Träger immobilisiert ist, bereitgestellt, umfassend die Schritte
(a) Auffinden des zu untersuchenden immobilisierten biologischen Materials unter Aufzeichnung dessen Position,
(b) Positionieren einer Vorrichtung zum Aufbringen des mindestens einen Reagenz über der in Schritt (a) aufgezeichneten Position des immobilisierten biologischen Materials und
(c) Aufbringen des mindestens einen Reagenz auf das immobilisierte biologische Material,
wobei auf dem Träger eine oder mehrere Positionen von gleichen oder unterschiedlichen immobilisierten biologischen Materialien aufgezeichnet werden.

Der Begriff "biologisches Material" kann jegliches in der Natur vorkommendes Material, insbesondere Zellen, Zellbestandteile wie Chromosomen, insbesondere Metaphasechromosomen, umfassen. Das biologische Material, wie z.B. Zellen oder Chromosomen, kann mit einem oder mehreren spezifischen Markern, beispielsweise Oberfächenmarkern wie markierten Antikörpern oder beispielsweise 23 unterschiedliche, unterscheidbar markierte Sonden zur Chromosomenanalyse, markiert sein. Der Begriff "Träger" kann jegliche Gegenstände, auf denen das biologische Material gehalten werden kann, insbesondere Objektträger für ein Mikroskop, umfassen. Der Begriff "immobilisiert" bedeutet, daß das biologische Material mit Hilfe der im Fachgebiet bekannten Methoden auf dem Träger verankert und/oder fixiert ist.

Das Auffinden des zu untersuchenden immobilisierten biologischen Materials in Schritt (a) des erfindungsgemäßen Verfahrens kann unter der Verwendung einer Abtasteinrichtung bzw. einer Vorrichtung zum gerichteten Abtasten wie z.B. einer Suchoptik, die z.B. ein Mikroskopobjektiv umfassen kann, erfolgen. In einer bevorzugten Ausführungsform wird in Schritt (b) des erfindungsgemäßen Verfahrens die Suchoptik gegen eine zum Aufbringen des mindestens einen Reagenz geeignete Vorrichtung ausgetauscht. Gemäß einer bevorzugten Ausführungsform des erfindungsgemäßen Verfahrens können z.B. in Verbindung mit einer Abtasteinrichtung und einer Bildverarbeitungseinheit, beispielsweise einer CCD-Kamera, während Schritt (a) eine oder mehrere Aufnahmen des gefundenen immobilisierten biologischen Materials aufgezeichnet werden. Vorzugsweise können die Aufnahmen zur Vorauswahl von geeignetem immobilisiertem biologischen Material zusammen angezeigt werden. Das Aufzeichnen der Position des immobilisierten biologischen Materials kann beispielsweise mit Hilfe einer Vorrichtung zum Abtasten der Position beispielsweise des Objektträgertisches eines Mikroskops in Verbindung mit einer rechnergestützten Aufzeichnung/Verarbeitung und Speicherung der Positionsdaten des immobilisierten biologischen Materials auf vorzugsweise digitalen Speichermedien wie Disketten, Festplatten, Wechselplatten, optische Speichermedien wie Compact Discs usw., erfolgen.

Eine (vorzugsweise automatisierte) Vorrichtung zum Aufbringen des mindestens einen Reagenz kann z.B. eine Pipette wie eine Mikroliterpipette umfassen.

In einer bevorzugten Ausführungsform der vorliegenden Erfindung umfaßt das Verfahren zum gezielten Aufbringen von Reagenzien auf immobilisierte Zellen oder Zellbestandteile zusätzlich den Schritt
(d) Positionieren einer Vorrichtung zum Aufbringen einer kleinflächigen lokalen Abdeckung über der in Schritt (a) aufgezeichneten Position des immobilisierten biologischen Materials.

Eine kleinflächige lokale Abdeckung kann z.B. ein Mikro-Deckgläschen umfassen.

Gemäß einer bevorzugten Ausführungsform des Verfahrens der vorliegenden Erfindung erfolgt das Aufbringen des mindestens einen Reagenz und/oder das Aufbringen der kleinflächigen lokalen Abdeckung automatisch. In einer weiteren bevorzugten Ausführungsform des erfindungsgemäßen Verfahrens ist die kleinflächige lokale Abdeckung mit in Reagenzien beschichtet oder benetzt.

Gemäß einer bevorzugten Ausführungsform des erfindungsgemäßen Verfahrens kann zwischen dem Auffinden des zu untersuchenden immobilisierten biologischen Materials unter Aufzeichnen dessen Position in Schritt (a) und dem Positionieren einer Vorrichtung zum Aufbringen des mindestens einen Reagenz über der in Schritt (a) aufgezeichneten Position des immobilisierten biologischen Materials in Schritt (b) wenigstens ein Zwischenschritt zum Behandeln, vorzugsweise Waschen des immobilisierten biologischen Materials eingefügt werden.

Gemäß einer weiteren Ausführungsform des erfindungsggemäßen Verfahrens ist es bevorzugt, daß eine Auswertung nach Schritt (c) oder (d) durch Positionieren einer Analysevorrichtung, umfassend beispielsweise eine Abtasteinrichtung oder eine Vorrichtung zum gerichteten Abtasten, die z.B. eine Vorrichtung für photometrische und/oder chemiluminometrische wie fluorometrische Messungen umfaßt, über der in Schritt (a) aufgezeichneten Position des immobilisierten biologischen Materials durchgeführt wird.

Gemäß einer weiteren Ausführungsform des erfindungsgemäßen Verfahrens umfaßt das mindestens eine Reagenz eine Nukleinsäure, ausgewählt aus DNA oder RNA, oder ein Polypetid. Der Begriff "Nukleinsäure" bedeutet native, halbsynthetische oder modifizierte Nukleinsäuremoleküle aus Desoxyribonukleotiden und/oder Ribonukleotiden und/oder modifizierten Nukleotiden wie Aminonukleotiden oder [a-S]-Triphosphatnukleotiden. Ein Polypeptid kann z.B. ein Protein wie einen Antikörper, der polyklonal oder monoklonal sein kann, umfassen.

Gemäß einer weiteren Ausführungsform des erfindungsgemäßen Verfahrens ist das mindestens eine Reagenz mit einer oder mehreren Markierungen versehen. Der Begriff "Markierung" bedeutet geeignete, direkt oder indirekt nachweisbare Atome oder Moleküle, die in die Moleküle des mindestens einen Reagenz eingebaut oder damit verbunden sind. Geeignete Markierungen sind beispielsweise solche, die an Nukleotide oder an Polypeptide, z.B. Antikörper, gekoppelte Fluoreszenzfarbstoffe und/oder Biotin und/oder Digoxigenin und/oder in Nukleotide oder in Polypeptide, z.B. Antikörper, eingebaute radioaktive Isotope umfassen. In einer bevorzugten Ausführungsform ist die Markierungsverbindung ein Fluoreszenzfarbstoff mit einem zur Selektion kleiner Substanzmengen ausreichendem Unterschied im Fluoreszenzverhalten der Emissionsspektren, wie z.B. Cumarine und Rhodamine und/oder der Fluoreszenzlebensdauer, wie z.B. Fluoreszenzisothiocyanate und Europium-Chelat-markierte und/oder Porphyrin-markierte Avidine.

Gemäß einer weiteren bevorzugten Ausführungsform des erfindungsgemäßen Verfahrens ist das mindestens eine Reagenz zur Hybridisierung oder Antigen/Antikörper-Reaktion oder Liganden/Protein-Reaktion befähigt. Der Begriff "Hybridisierung" insbesondere "*In-situ*-Hybridisierung" bedeutet die Anlagerung einer synthetisch erzeugten und mit biologischen, physikalischen oder chemischen Markierungen zur Detektion versehenen DNA/RNA-Sonde als Reagenzmolekül an native, in der Natur vorkommende DNA/RNA-Sequenzen, wobei die Anlagerung durch Denaturierung und Renaturierung der entsprechenden Nukleinsäuren erreicht wird. Selbstverständlich enthalten diese DNA/RNA-Sonden mindestens ein zur Hybridisierung an eine DNA/RNA-Sequenz der Moleküle des immobilisierten biologischen Materials wie einem Chromosom befähigten Sequenzabschnitt. Dieser Sequenzabschnitt weist einen spezifischen, einzeln vorliegenden, vorzugsweise 100 bis 1.000 Basenpaare langen Sequenzbereich des Reagenzmoleküls auf, der sich an einen komplementären Bereich des Moleküls des immobilisierten biologischen Materials bei einer geeigneten Temperatur, vorzugsweise 50°C oder weniger, und bei einer geeigneten Salzkonzentration, die vorzugsweise 50 bis 300 mmol/l einwertige lonen und 0 bis 10 mmol/l zweiwertige Ionen enthält, unter Ausbildung von Wasserstoffbrücken anlagert.

Im Rahmen des vorliegenden verfahrens kann zur Untersuchung von immobilisiertem biologischem Material nach einem der vorstehend definierten Verfahren ein diagnostischer Kit verwendet werden, enthaltend kleinflächige lokale Abdeckungen, die vorzugsweise Mikro-Deckgläser umfassen, zur Kontakthybridisierung, auf denen die Reagenzien fixiert sind.

Im Fall der Metaphasenanalyse wird das erfindungsgemäße Verfahren so durchgeführt, daß eine Vorrichtung zum gezielten Relokalisieren zuvor aufgesuchter geeigneter Metaphasechromosomen verwendet wird, so daß es einfach und schnell und damit routinetauglich eingesetzt werden kann. Die Positionen von manuell oder automatisch aufgefundenen Metaphasechromosomen werden gespeichert. Anschließend werden die gefundenen Metaphasechromosomen automatisch unter eine Pipette mit der Sondenlösung positioniert, um die Hybridisierungslösung in kleiner Menge gezielt aufzubringen. In einem weiteren Schritt können die gefundenen Metaphasechromosomen unter eine weitere Pipette oder geeignete Vorrichtung positioniert werden, um ein kleines Deckglas automatisch aufzubringen.

Der Materialverbrauch, beispielsweise bei Hybridisierungen von Metaphasechromosomen mit DNA-Sonden, kann mit Hilfe der erfindungsgemäßen Verfahren um einen Faktor 10 oder mehr reduziert werden. Dies ist insbesondere bei der Tumorzytogenetik von Bedeutung, wo zum einen die Zahl der Metaphasen pro Objektträger gering ist, und andererseits die Möglichkeit einer Vorauswahl von Metaphasen - beispielsweise entsprechend einer Ausführungsform der vorliegenden Erfindung über eine Anzeige der Aufnahmen der automatisch gefundenen Metaphasen -, die eine für die Auswertung geeignete Chromosomenqualität aufweisen, vor Aufbringen der DNA-Sonden von besonderem Interesse ist.

Die erfindungsgemäßen Verfahren ist anwendbar auf zuvor mit geeigneten Markern markierte Interphasezellen, die bestimmte Eigenschaften aufweisen und mit geringer Frequenz im Präparat vorkommen, wie z.B. Tumorzellen beim Nachweis von minimaler Resterkrankung oder fetale Zellen im mütterlichen Blut.

## Patentansprüche

1. Verfahren zum gezielten Aufbringen von mindestens einem Reagenz auf biologisches Material, das auf einem Träger immobilisiert ist, umfassend die Schritte
(a) Auffinden des zu untersuchenden immobilisierten biologischen Materials unter Aufzeichnung dessen Position,
(b) Positionieren einer Vorrichtung zum Aufbringen des Reagenz über der in Schritt (a) aufgezeichneten Position des immobilisierten biologischen Materials und
(c) Aufbringen des Reagenz auf das immobilisierte biologische Material,
wobei auf dem Träger eine oder mehrere Positionen von gleichen oder unterschiedlichen immobilisierten biologischen Materialien aufgezeichnet werden.

2. Verfahren nach Anspruch 1, wobei das Auffinden des zu untersuchenden immobilisierten biologischen Materials in Schritt (a) mit Hilfe einer Suchoptik durchgeführt wird und in Schritt (b) die Suchoptik gegen eine zum Aufbringen des Reagenz geeignete Vorrichtung ausgetauscht wird.

3. Verfahren nach Anspruch 1 oder 2, das zusätzlich den Schritt
(d) Positionieren einer Vorrichtung zum Aufbringen einer kleinflächigen lokalen Abdeckung über der in Schritt (a) aufgezeichneten Position des immobilisierten biologischen Materials
umfaßt.

4. Verfahren nach einem der Ansprüche 1 bis 3, wobei das Aufbringen des Reagenz automatisiert ist.

5. Verfahren nach Anspruch 3 oder 4, wobei die kleinflächige lokale Abdekkung mit dem Reagenz beschichtet oder benetzt ist.

6. Verfahren nach einem der Ansprüche 3 bis 5, wobei das Aufbringen der kleinflächigen lokalen Abdeckung automatisiert ist.

7. Verfahren nach einem der Ansprüche 1 bis 6, wobei während Schritt (a) eine oder mehrere Aufnahmen des aufgefundenen immobilisierten biologischen Materials aufgezeichnet werden.

8. Verfahren nach Anspruch 7, wobei die Aufnahmen zur Vorauswahl von geeignetem immobilisiertem biologischen Material zusammen angezeigt werden.

9. Verfahren nach einem der Ansprüche 1 bis 8, wobei zwischen dem Auffinden des zu untersuchenden immobilisierten biologischen Materials unter Aufzeichnung dessen Position in Schritt (a) und dem Positionieren einer Vorrichtung zum Aufbringen des mindestens einen Reagenz über der in Schritt (a) aufgezeichneten Position des immobilisierten biologischen Materials in Schritt (b) wenigstens ein Zwischenschritt zum Behandeln, vorzugsweise Waschen, des immobilisierten biologischen Materials eingefügt ist.

10. Verfahren nach einem der Ansprüche 1 bis 9, wobei eine Auswertung nach Schritt (c) oder (d) durch Positionieren einer Analysevorrichtung über der in Schritt (a) aufgezeichneten Position des immobilisierten biologischen Materials durchgeführt wird.

11. Verfahren nach einem der Ansprüche 1 bis 10, wobei das aufzufindende immobilisierte biologische Material Zellen umfaßt.

12. Verfahren nach Anspruch 11, wobei die Zellen mit spezifischen Markern markiert sind.

13. Verfahren nach einem der Ansprüche 1 bis 10, wobei das aufzufindende immobilisierte biologische Material Metaphasechromosomen umfaßt.

14. Verfahren nach einem der Ansprüche 1 bis 13, wobei das Reagenz eine Nukleinsäure, ausgewählt aus DNA oder RNA, oder ein Polypeptid umfaßt.

15. Verfahren nach einem der Ansprüche 1 bis 14, wobei das Reagenz mit einer oder mehreren, gleichen oder unterschiedlichen Markierungen versehen ist.

16. Verfahren nach Anspruch 15, wobei die Markierung des Reagenz mindestens einen Fluoreszenzfarbstoff umfaßt.

17. Verfahren nach Anspruch 14 bis 16, wobei das Reagenz zur Hybridisierung oder Antigen/Antikörper-Reaktion oder Liganden/Protein-Reaktion befähigt ist.

## Claims

1. Method for selectively applying at least one reagent to biological material which is immobilised on a support, comprising the steps of
(a) detecting the immobilised biological material to be investigated while recording its position,
(b) positioning a device for applying the reagent over the position, which was recorded in step (a) of the immobilised biological material, and
(c) applying the reagent to the immobilised biological material,
**characterized in that** one or more positions of identical or different immobilised biological materials is/are recorded on the support.

2. Method according to Claim 1, **characterized in that** the immobilised biological material to be investigated is detected in step (a) using a screening lens system and, in step (b), the screening lens system is replaced with a device which is suitable for applying the reagent.

3. Method according to Claim 1 or 2 which additionally comprises the step of
(d) positioning a device for applying a small-area local cover over the position, which was recorded in step (a), of the immobilised biological material.

4. Method according to one of Claims 1 to 3, **characterized in that** the application of the reagent is automated.

5. Method according to Claim 3 or 4, **characterized in that** the small-area local cover is coated or wetted with the reagent.

6. Method according to one of Claims 3 to 5, **characterized in that** the application of the small-area local cover is automated.

7. Method according to one of Claims 1 to 6, **characterized in that** one or more photographs of the immobilised biological material which has been detected is/are recorded during step (a).

8. Method according to Claim 7, **characterized in that** the photographs are displayed together for the purpose of preselecting suitable immobilised biological material.

9. Method according to one of Claims 1 to 8, **characterized in that** at least one intermediate step for treating, preferably washing, the immobilised biological material is inserted between detecting, in step (a), the immobilised biological material to be investigated, while recording its position, and positioning, in step (b), a device for applying at least the one reagent over the position, which was recorded in step (a), of the immobilised biological material.

10. Method according to one of Claims 1 to 9, **characterized in that** an assessment is carried out after step (c) or (d) by positioning an analytical device over the position, which was recorded in step (a), of the immobilised biological material.

11. Method according to one of Claims 1 to 10, **characterized in that** the immobilised biological material to be detected comprises cells.

12. Method according to Claim 11, **characterized in that** the cells are labelled with specific labels.

13. Method according to one of Claims 1 to 10, **characterized in that** the immobilised biological material to be detected comprises metaphase chromosomes.

14. Method according to one of Claims 1 to 13, **characterized in that** the reagent comprises a nucleic acid, selected from DNA or RNA, or a polypeptide.

15. Method according to one of Claims 1 to 14, **characterized in that** the reagent is provided with one or more identical or different labels.

16. Method according to Claim 15, **characterized in that** the reagent label comprises at least one fluorescent die.

17. Method according to Claims 14 to 16, **characterized in that** the reagent is capable of hybridisation or of an antigen/antibody reaction or of a ligand/protein reaction.

## Revendications

1. Procédé d'application ciblé d'au moins un réactif sur une matière biologique qui est immobilisée sur un support, comprenant les étapes qui consistent :
(a) à détecter la matière biologique immobilisée à examiner en notant sa position,
(b) à positionner un dispositif d'application du réactif au-dessus de la position notée dans l'étape (a) de la matière biologique immobilisée et
(c) à appliquer le réactif sur la matière biologique immobilisée,
en notant sur le support une ou plusieurs positions de matières biologiques immobilisées identiques ou différentes.

2. Procédé suivant la revendication 1, dans lequel la détection de la matière biologique immobilisée à examiner dans l'étape (a) est effectuée à l'aide d'un instrument optique d'exploration et, dans l'étape (b), l'instrument optique d'exploration est remplacé par un dispositif qui convient pour l'application du réactif.

3. Procédé suivant la revendication 1 ou 2, qui comprend en outre l'étape consistant :
(d) à positionner un dispositif d'application d'un cache local de faible surface au-dessus de la position, notée dans l'étape (a), de la matière biologique immobilisée.

4. Procédé suivant l'une des revendications 1 à 3, dans lequel l'application du réactif est automatisée.

5. Procédé suivant la revendication 3 ou 4, dans lequel le cache local de faible surface est revêtu ou imprégné du réactif.

6. Procédé suivant l'une des revendications 3 à 5, dans lequel l'application du cache local de faible surface est automatisée.

7. Procédé suivant l'une des revendications 1 à 6, dans lequel un ou plusieurs clichés de la matière biologique immobilisée détectée peuvent être enregistrés pendant l'étape (a).

8. Procédé suivant la revendication 7, dans lequel les clichés sont affichés ensemble en vue de la présélection de matière biologique immobilisée appropriée.

9. Procédé suivant l'une des revendications 1 à 8, dans lequel au moins une étape intermédiaire de traitement, de préférence par lavage, de la matière biologique immobilisée, est intercalée entre l'étape (a) de détection de la matière biologique immobilisée à examiner avec notation de sa position, et l'étape (b) de positionnement d'un dispositif d'application d'au moins un réactif au-dessus de la position, notée dans l'étape (a), de la matière biologique immobilisée.

10. Procédé suivant l'une des revendications 1 à 9, dans lequel une évaluation est effectuée après l'étape (c) ou (d) par positionnement d'un dispositif d'analyse au-dessus de la position, notée dans l'étape (a), de la matière biologique immobilisée.

11. Procédé suivant l'une des revendications 1 à 10, dans lequel la matière biologique immobilisée à examiner comprend des cellules.

12. Procédé suivant la revendication 11, dans lequel les cellules sont marquées avec des marqueurs spécifiques.

13. Procédé suivant l'une des revendications 1 à 10, dans lequel la matière biologique immobilisée à examiner comprend des chromosomes en métaphase.

14. Procédé suivant l'une des revendications 1 à 13, dans lequel le réactif comprend un acide nucléique choisi entre un ADN ou un ARN, ou un polypeptide.

15. Procédé suivant l'une des revendications 1 à 14, dans lequel le réactif est doté d'un ou plusieurs marquages identiques ou différents.

16. Procédé suivant la revendication 15, dans lequel le marquage du réactif comprend au moins un colorant fluorescent.

17. Procédé suivant les revendications 14 à 16, dans lequel le réactif est capable d'une hybridation ou d'une réaction antigène/anticorps ou d'une réaction ligand/protéine.
